# EUROPEAN PATENT SPECIFICATION

(11) **EP 2 336 773 B1**
(45) Date of publication and mention of the grant of the patent: **29.05.2013**
(21) Application number: 08800529.3
(22) Date of filing: 26.08.2008
(51) Int. Cl.: G01N 33/53, G01N 33/558

(54) **A FLUID TEST CHIP BASE PLATE**
FLUIDTESTCHIP-GRUNDPLATTE
PLAQUE DE BASE DE PUCE DE TEST DE FLUIDE

(43) Date of publication of application: 22.06.2011
(73) Proprietor: Actherm Inc., Hsinchu 30078 (TW)
(72) Inventor: HSIEH, Wen-Pin, Taiwan 352 (CN); WU, Yi-Jen, Taiwan 302 (CN)
(74) Representative: Lang, Christian
(86) International application number: PCT/CN2008/001531
(87) International publication number: WO 2010/022537

(56) References cited:
- WO-A1-2004/086042
- WO-A1-2006/047869
- CN-A- 1 139 982
- CN-A- 1 407 339
- CN-A- 1 519 563
- CN-A- 1 830 390
- US-A- 4 828 801
- US-A1- 2003 108 949

## Description

### BACKGROUND OF THE INVENTION

### 1. Technical Field

The present invention relates to a substrate, and more particularly, to a substrate of an analytical strip for biochemical or immunological assays.

### 2. Description of Related Art

Conventional analytical strips used in biochemical or immunological assays usually have a substrate or a baseboard provided with a channel or a microfluidic channel. While such channel is typically bordered by a non-absorbent material, and the viscosity of the fluid sample to be analyzed is usually high for the sample is mainly composed of proteins or carbohydrates, part of the fluid sample tends to adhere to the surface of the channel and will not be reacted. Such scenario, if happens, will not only disadvantageously cause the waste of the fluid sample to be analyzed, but also will adversely affects the accuracy of quantifying assays.

In addition, the conventional analytical strip may facilitate the flow of the fluid sample by microfluidic channels so that the fluid sample will be delivered via the capillary force exerted by the structures of such channels to the reaction area. Another alternative approach to deliver the fluid sample involves applying a driving force, such as by a pressurizing means, at the time the fluid sample is introduced into the channel so that the fluid sample is propelled to the reaction area through the channel. However, either one of the aforementioned approaches tends to cause air bubbles occurring after the fluid sample is introduced into the channel. These bubbles, either large or small, will block the channel and result in inaccurate analyzing results.

In addition, the manufacturing process of the channels or microfluidic channels on the current substrates is usually involves molding, injection forming or imprinting. Consequently, the analytical strips comprising those abovementioned substrates have to be made of high-priced micro-injection molds manufactured by using micro-machining or LIGA (abbreviation of "Lithographie GalVanoformung Abformung", or "Lithography Electroforming Micro Molding" in english) technique. The micro-injection mold used in the manufacturing process tends to wear out rapidly, which results in the relatively high cost.

WO 2004/086042 A1 describes a multiple channel test device, which includes a plurality of channels formed by removing portions, where no fluid is allowed to flow, from a porous material. The porous material forming the flowing passages are provided with binding agents immobilized in the porous material. However, the production of this test device is complex and time consuming.

US 2003/0108949 Al describes a filtration-based micro array chip, which is composed of a substrate and a nitrocellulose film disposed thereon, wherein the film further includes analyte-specific capture molecules. However, for the reason of analysis a high amount of fluid sample is necessary.

Thus, starting from the cited prior art, it is the object of the present application to provide a test device which is easy to produce and assures a secure analysis of a fluid already at a minimal amount of fluid sample.

### SUMMERY OF THE INVENTION

The above-mentioned object is solved by the substrate of an analytical strip according to claim 1. Advantageous improvements of the present invention are subject matter of the dependant claims.

In an attempt to overcome the recited drawbacks of the conventional analytical strips, the present invention provides a substrate of an analytical strip having a channel provided concavely on an upper surface thereof. The channel comprises a first area for receiving a fluid sample, a second area for delivering the fluid sample, and a third area where the fluid sample reacts. These areas are connected sequentially. The substrate is characterized in that nitrocellulose layers are formed at each bottom of both the second and the third area, and the conformation of the nitrocellulose layers is a hollow matrix. In addition, the nitrocellulose layer of the second area has an average thickness that is not greater than the thickness of the nitrocellulose layer of the third area.

The advantages of the an analytical strip of this invention are as below:
1) because the analytical strip has a thin absorptive nitrocellulose layer on the bottom of channel and the thin absorptive nitrocellulose layers act as sample delivering and/or separating function, the channel thus has lower residual of samples in contrast to the traditional microfluidic channel. And low volume of samples needed for multi-analytes detection in a test is realized.
2) because the analytical strip has absorptive nitrocellulose layers and has a constant volumetric absorptive capacity, thus the analytical strip allows a quantitative assay to be conducted via controlling the volume of the nitrocellulose
3) Because the absorptive nitrocellulose layers with a hollow-matrix configuration, which is capable of destroying the air bubbles in the fluid sample when the fluid sample flows through the hollow matrix, as well as preventing the bubbles from blocking the channel or the microfluidic channel of the substrate, an accurate result of the quantitative assay could be assured.

### BRIEF DESCRIPTION OF THE DRAWINGS

The invention as well as a preferred mode of use, further objects and advantages thereof will be best understood by reference to the following detailed description of illustrative embodiments when read in conjunction with the accompanying drawings, wherein:
Fig. 1A is a perspective view of a substrate of an analytical strip according to the first embodiment of the present invention;
Fig. 1B is a cross-sectional view of the substrate of the analytical strip according to the first embodiment of the present invention;
Fig. 2A is a perspective view of a substrate of an analytical strip according to the second embodiment of the present invention; and
Fig. 2B is a cross-sectional view of the substrate of the analytical strip according to the second embodiment of the present invention.

### DETAILED DESCRIPTION OF THE PREFERRED EMBODIMENTS

While the present invention proposes a substrate of an analytical strip, the physical and chemical principles, as well as solution applying techniques it employs are known to one skilled in the art and need not be discussed at any length herein. Meanwhile, the accompanying drawings referred to in the following description are provided for illustrative purposes and need not to be made to scale.

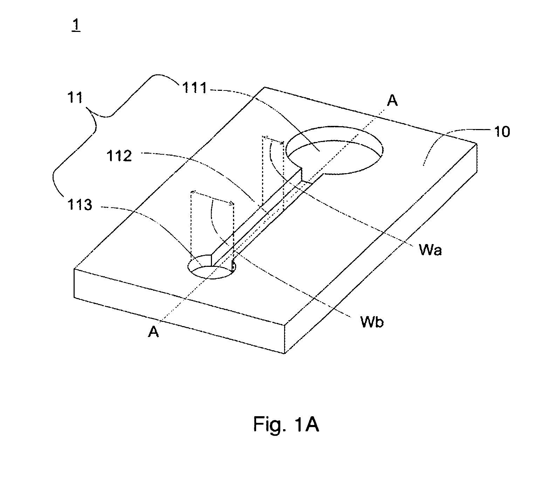

Fig. 1A is a perspective view of a substrate of an analytical strip according to the first embodiment of the present invention. The substrate 1 of the analytical strip has an upper surface 10 concavely provided with a channel 11. The channel 11 includes a first area 111, a second area 112 and a third area 113 that are connected sequentially. The first area 111 is to receive a fluid sample to be analyzed. The fluid sample is introduced to the first area 111 and then delivered by the second area 112 to the third area 113 where the analytes of the fluid sample are reacted. In a preferred mode of the present invention, the substrate 1 is made of a biocompatible material.

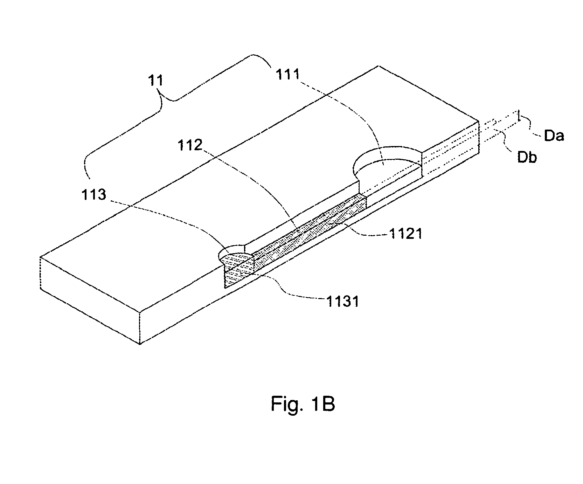

Please refer to Fig. 1B, which is a cross-sectional view of the substrate 1 taken along Line A-A of Fig. 1A. The substrate 1 is characterized in that the nitrocellulose layers 1121 and 1131 are formed respectively at both bottoms of the second area 112 and the third area 113. Each of the nitrocellulose layers 1121 and 1131 has a hollow-matrix conformation. The porous hollow matrix serves to absorb the fluid sample coming from the first area 111 and the analytes in the fluid sample can react with the pre-embedded reagents in the nitrocellulose layer 1131. Since the nitrocellulose layers 1121 and 1131 are absorptive, the channel 11 thus has lower residual of samples in contrast to the traditional microfluidic channel, and low volume of samples needed for multi-analytes detection in a test is realized. In addition, when the fluid sample flows through the nitrocellulose layers 1121 and 1131, the hollow-matrix conformation will destroy the air bubbles in the fluid sample, thereby preventing the bubbles from blocking the channel 11. In addition, the nitrocellulose layer 1121 of the second area 112 has an average thickness Da which is not greater than the average thickness Db of the nitrocellulose layer 1131 of the third area 113, namely that Da is smaller than or equal to Db. Furthermore, in order to reduce the volume of the fluid sample required, the width Wa of the second area 112 and the width Wb of the third area 113 (shown in Fig. 1A) are both preferably 0.3 mm at least.

The nitrocellulose layers 1121 and 1131 are formed as the following steps.

The nitrocellulose layers 1121 and 1131 are formed as the following steps. Firstly, a nitrocellulose powder is mixed with an organic solvent containing esters and ketones to form a nitrocellulose solution. The nitrocellulose solution is then applied to the bottoms of the second and third areas 112 and 113 via a casting process. After drying, the nitrocellulose layers 1121 and 1131 are formed respectively at the bottoms of the second area 112 and third area 113. For a better result of the casting process, the surface roughness (Ra) of the channel 11 preferably ranges from about 3µm to about 50µm.

As previously described, after drying the nitrocellulose solution applied to the bottoms of the second areas 112 and third area 113 forms the nitrocellulose layers 1121 and 1131 which both have the hollow-matrix conformation. In order to obtain a hollow matrix with a better structure, the nitrocellulose powder preferably has a volume that is about one ninth of the volume of the organic solvent containing esters and ketones. Because each volumetric unit of nitrocellulose has constant absorptive capacity, before casting the required volume of the nitrocellulose solution can be derived from the desired volume of the fluid sample to be adsorbed and analyzed. As a result, the required volume of the fluid sample to be analyzed is fixedly set, and the resultant analytical strip is suitable for an assay in a small volume. area 211 for receiving a fluid sample to be analyzed, a second area 212 for delivering the fluid sample, a third area 213 and a fourth area 214. These four areas 211, 212, 213 and 214 are connected sequentially. The fluid sample is introduced to the first area 211 and then delivered via the second area 212 to the third area 213 where the analytes of the fluid sample are reacted.

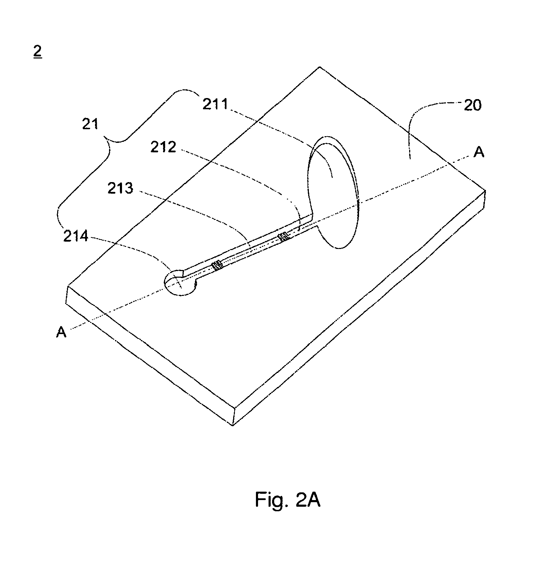

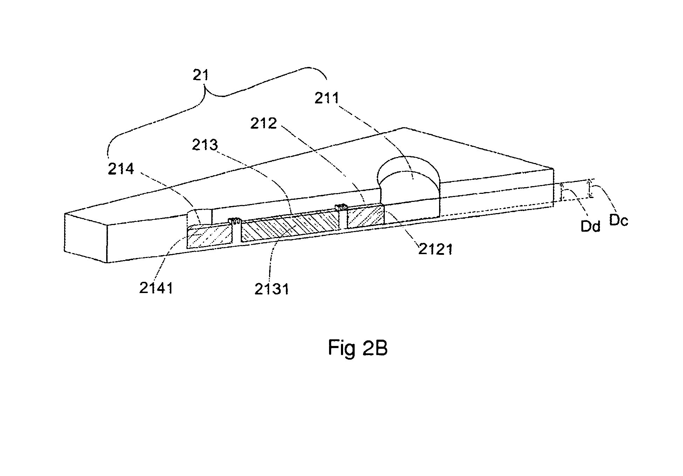

Please refer to Fig. 2B, which is a cross-sectional view of the substrate 2 taken along Line A-A of Fig. 2A. Nitrocellulose layers 2121 and 2131 are formed at bottoms of the second area 212 and the third area 213 respectively. In addition, the nitrocellulose layer 2121 of the second area 212 has an average thickness Dc that equals to the average thickness Dd of the nitrocellulose layer 2131 of the third area 213. Similar to the second and third areas 212 and 213, a nitrocellulose layer 2141 is also formed at the bottom of the fourth area 214 and also has a hollow-matrix conformation for accommodating the excess fluid sample. The nitrocellulose layer 2141 at the bottom of the fourth area 214 is made in the same way as the nitrocellulose layers 2121 and 2131. Namely, the nitrocellulose layers 2121, 2131 and 2141 are formed by casting a nitrocellulose solution onto the bottoms of the second, third and fourth areas 212, 213, and 214, followed by a drying process.

In addition, in the second embodiment, all the structures, dimensions and connective relationships of the first, second and third areas, the preferred material of the substrate, the surface roughness of the channel, the conformation and forming method of the nitrocellulose layers, the preferred compositions of the nitrocellulose solution and the preferred ratio therebetween are all similar to those described in the first embodiment and will not to be described herein in further detail.

The present invention has been described with reference to the preferred embodiments and it is understood that the embodiments are not intended to limit the scope of the present invention. Moreover, as the contents disclosed herein should be readily understood and can be implemented by a person skilled in the art, all equivalent changes or modifications which do not depart from the concept of the present invention should be encompassed by the appended claims.

## Claims

1. A substrate (1) of an analytical strip, having a channel (11) provided concavely on an upper surface (10) thereof, wherein said channel (11) comprises a first area (111) for receiving a fluid sample, a second area (112), and a third area (113) and these three areas are connected sequentially, the substrate (1) being **characterized in that**:
at the bottom thereof, each of the second and the third area (112, 113) comprises a nitrocellulose layer (1121, 1131) having a hollow-matrix conformation, wherein the second area (112) is for delivering the fluid sample and the third area is where the fluid sample reacts, the nitrocellulose layer (1121) of the second area (112) comprises an average thickness (Da) which is smaller than that (Db) of the nitrocellulose layer (1131) of the third area (113), and each of the second area (112) and the third area (113) has a width (Wa, Wb) of at least 0.3 mm;
wherein:
the channel (11) has a surface roughness ranging from 3µm to 50µm, both nitrocellulose layers (1121, 1131,) of the second and third areas (112, 113) are formed by casting a nitrocellulose solution onto both bottoms of the second and third areas (112, 113), and then being followed by a drying process;
the nitrocellulose solution is a mixture of a nitrocellulose powder and an organic solvent containing ester and ketones; and
the nitrocellulose powder is mixed with the solvent containing esters and ketones at a volumetric ratio of 1:9.

2. The substrate (1) of Claim 1, wherein the substrate (1) is made of a biocompatible material.

3. The substrate (2) of Claim 1, wherein the channel (21) further comprises a fourth area (214) having a nitrocellulose layer (2141) which is formed at the bottom thereof and also has a hollow-matrix conformation for accommodating excess of the fluid sample.

4. The substrate (2) of Claim 3, wherein all the nitrocellulose layers (2121,2131,2141) of the second, the third and the forth areas (212,213,214) are formed by casting a nitrocellulose solution onto each bottom of the second, the third and the fourth areas (212,213,214), respectively, and then being followed by a drying process.

5. The substrate (2) of Claim 4, wherein the nitrocellulose solution is a mixture of a nitrocellulose powder and an organic solvent containing esters and ketones.

6. The substrate (2) of Claim 5, wherein the nitrocellulose powder is mixed with the solvent containing esters and ketones at a volumetric ration of 1:9.

## Patentansprüche

1. Substrat (1) eines Analysestreifens, das einen Kanal (11) umfasst, der konkav an dessen oberen Oberfläche (10) vorgesehen ist, Wobei der Kanal (11) einen ersten Bereich (111) zum Aufnehmen einer Flüssigkeitsprobe, einen zweiten Bereich (112) und einen dritten Bereich (113) umfasst und diese drei Bereiche der Reihe nach miteinander verbunden sind, und wobei das Substrat (1) **dadurch gekennzeichnet ist, dass**:
an dessen Boden sowohl der zweite als auch der dritte Bereich (112, 113) eine Nitrocelluloseschicht (1121, 1131) umfassen, die eine hohle Matrixgestalt aufweist, wobei der zweite Bereich (112) für das Transportieren der Flüssigkeitsprobe ist und der dritte Bereich der ist, in dem die Flüssigkeitsprobe reagiert, wobei die Nitrocelluloseschicht (1121) des zweiten Bereichs (112) eine durchschnittliche Dicke (Da) umfasst, die kleiner ist als diejenige (Db) der Nitrocelluloseschicht (1131) des dritten Bereichs (113), wobei sowohl der zweite Bereich (112) als auch der dritte Bereich (113) eine Breite (Wa, Wb) von mindestens 0.3 mm aufweisen;
wobei:
der Kanal (11) eine Oberflächenrauheit im Bereich von 3 µm bis 50 µm aufweist, wobei sowohl die Nitrocelluloseschicht (1121, 1131) des zweiten als auch des dritten Bereichs (112, 113) durch Gießen einer Nitrocelluloselösung auf beide Böden der zweiten als auch der dritten Schicht (112, 113) gebildet ist, gefolgt von einem Trocknungsprozess;
wobei die Nitrocelluloselösung eine Mischung aus Nitrocellulosepulver und einer organischen Lösung ist, die Esther und Ketone enthält; und wobei
das Nitrocellulosepulver mit der Lösung, die Esther und Ketone enthält, mit einem Volumenverhältnis von 1:9 gemischt wird.

2. Substrat (1) nach Anspruch 1, wobei das Substrat (1) aus einem biokompatiblen Material hergestellt ist.

3. Substrat (2) nach Anspruch 1, wobei der Kanal (21) ferner einen vierten Bereich (214) umfasst, der eine Nitrocelluloseschicht (2141) aufweist, die an dessen Boden ausgebildet ist und die auch eine hohle Matrixgestalt zum Aufnehmen von überschüssigen Flüssigkeitsproben aufweist.

4. Substrat (2) nach Anspruch 3, wobei alle Nitrocelluloseschichten (2121, 2131, 2141) der zweiten, der dritten und der vierten Bereiche (212, 213, 214) durch Gießen einer Nitrocelluloselösung auf den jeweiligen Boden des zweiten, des dritten und des vierten Bereichs (212, 213, 214) ausgebildet werden, gefolgt von einem Trocknungsprozess.

5. Substrat (2) nach Anspruch 4, wobei die Nitrocelluloselösung eine Mischung aus Nitrocellulosepulver und einer organischen Lösung ist, die Esther und Ketone enthält.

6. Substrat (2) nach Anspruch 5, wobei das Nitrocellulosepulver mit der Lösung, die Esther und Ketone enthält, mit einem Volumenverhältnis von 1:9 gemischt wird.

## Revendications

1. Substrat (1) de bande analytique ayant un canal (11) prévu de manière concave sur une surface supérieure (10) de celui-ci, ledit canal (11) comprenant une première zone (111) pour recevoir un échantillon de fluide, une seconde zone (112) et une troisième zone (113) et ces trois zones étant reliées de manière séquentielle, le substrat étant **caractérisé en ce qu'**au fond de celui-ci, chacune de la seconde zone et de la troisième zone (112, 113) comprend une couche de nitrocellulose (1121, 1131) avant une conformation de matrice creuse, la seconde zone (112) étant pour délivrer l'échantillon de fluide et la troisième zone est où l'échantillon de fluide réagit, la couche de nitrocellulose (1121) de la seconde zone (112) comprend une épaisseur moyenne (Da) qui est plus petite que celle (Db) de la couche de nitrocellulose (1131) de la troisième zone (113) et chacune des zones, la seconde zone (112) et la troisième zone (113) a une largeur (Wa, Wb) d'au moins 0,3 mm,
le canal (11) ayant une rugosité de surface de l'ordre de 3 µm à 50 µm, les deux couches de nitrocellulose (1121, 1131) de la seconde et de la troisième zones (112, 113) étant formés en coulant une solution de nitrocellulose sur les deux fonds de la seconde et de la troisième zones, ceci étant ensuite suivi par un processus de séchage,
la solution de nitrocellulose étant un mélange de poudre de nitrocellulose et de solvant organique contenant de l'ester et des cétones et
la poudre de nitrocellulose étant mélangée au solvant contenant des esters et des cétones dans un rapport volumétrique d'1 à 9.

2. Substrat (1) selon la revendication 1, le substrat (1) étant en un matériau biocompatible.

3. Substrat (2) selon la revendication 1, le canal (21) comprenant de plus une quatrième zone (214) ayant une couche de nitrocellulose (2141) qui est formée au fond de celle-ci et également une conformation de matrice creuse pour loger l'excédent d'échantillon de fluide.

4. Substrat (2) selon la revendication 3, toutes les couches de nitrocellulose (2121, 2131, 2141) de la seconde, de la troisième et de la quatrième zone (212, 213, 214) étant formées en coulant une solution de nitrocellulose sur chaque fond de la seconde, de la troisième et de la quatrième zone (212, 213, 214) respectivement et ceci étant ensuite suivi par un processus de séchage.

5. Substrat (2) selon la revendication 4, la solution de nitrocellulose étant un mélange de poudre de nitrocellulose et d'un solvant organique contenant des esters et des cétones.

6. Substrat (2) selon la revendication 5, la poudre de nitrocellulose étant mélangée avec le solvant contenant des esters et des cétones dans un rapport volumétrique d'1 à 9.
